(19) Europäisches Patentamt
European Patent Office
Office européen des brevets

(11) Publication number: **0 363 224 A1**

# (12) EUROPEAN PATENT APPLICATION

(21) Application number: 89310283.0

(22) Date of filing: 06.10.89

(51) Int. Cl.5: A61K 7/16 , A61K 7/24 , A61K 31/19

(30) Priority: 07.10.88 US 254587

(43) Date of publication of application: 11.04.90 Bulletin 90/15

(84) Designated Contracting States: AT BE CH DE ES FR GB GR IT LI LU NL SE

(71) Applicant: BLOCK DRUG COMPANY INC.
257 Cornelison Avenue
Jersey City New Jersey(US)

(72) Inventor: Rosenthal, Murray W.
32 Jensen Street
E. Brunswick New Jersey(US)
Inventor: Yeh, Kuo-Chen
210 Golf Edge
Westfield New Jersey(US)
Inventor: Synodis, Joseph
66 Rotary Drive
Summit New Jersey(US)

(74) Representative: Ablewhite, Alan James et al
MARKS & CLERK 57/60 Lincoln's Inn Fields
London WC2A 3LS(GB)

## (54) Treatment of periodontal disease.

(57) Described is the use of a (3-benzoylphenyl) alkanoic acid derivative applied to the gingiva of humans for the purpose of inhibiting the formation of endoperoxides, thromboxanes, leukotrienes, prostaglandins and related tissue-destructive end-products of arachidonic acid metabolism, for the prophylaxis or treatment of periodontal disease, preferably in a mucosal-tenacious vehicle which is selected from the groups of semi-solid pastes, gels, ointments, liquids or films.

EP 0 363 224 A1

# TREATMENT OF PERIODONTAL DISEASE

## BACKGROUND OF THE INVENTION

Periodontal disease is a more insidious and dangerous threat to human tooth integrity and oral health than dental caries or any other known pathologic condition of the mouth. It develops so gradually that the affected person is frequently unaware of the destructive forces taking place, and usually it is not until tissues develop a high degree of inflammation and start to bleed, tooth loosening occurs and deep pocket formation develops, that any corrective remedies are sought, frequently too late to save the teeth. Almost 40% of children 6 to 11 years old have periodontal disease in some form and the percentage of those afflicted increases with age until about in the 6th decade, about 3 out of 4 adults who still retain their natural dentition have periodontal disease in some stage of progression. The disease is not limited to affluent societies and in fact, in those parts of the world where nutrition and oral hygiene are below minimally acceptable levels, the incidence, age of onset and severity of periodontal disease are so marked as to result in total tooth loss early in life.

The causes of periodontal disease are extremely complex and still not completely understood. Oral bacteria in dental plaque are believed to play a major role in the multifaceted destructive process. The human oral cavity supports a dense and varied spectrum of microorganisms, localized in three main foci, the dorsal area of the tongue, the gingival crevice and the adhesive and tenacious plaque on teeth in areas not adequately cleansed by mastication or effective oral hygiene.

Implication of plaque as a probable causative factor in the development of gingival tissue inflammation was postulated by Löe et al. in 1965 (Löe, H., Theilade, E. and Jensen, S. "Experimental Gingivitis in Man," J. Periodontol. 36:177, 1965) and by Theilade et al. in 1966 (Theilade, E., Wright, W., Jensen, S. and Löe, H. "Experimental Gingivitis in Man: II. A Longitudinal and Bacteriological Investigation", J. Periodont. Res. 1:1, 1966). These investigators demonstrated that subjects who refrained from oral hygiene procedures for 2-3 weeks developed both complex and heavy plaque deposits and demonstratable gingival tissue inflammation. Re-institution of oral hygiene procedures rapidly reduced plaque deposits and reversed the tissue inflammatory process.

Dental plaque consists principally of microbial masses, with approximately 250 million individual bacterial cells per mg. of plaque wet weight. From 100-200 mg. of total plaque can be recovered from the teeth of periodontal disease sufferers. The number of different species of microorganisms which can be identified in normal and disease-associated plaque is astonishingly high and includes S. sanguis, A. viscosus, A. naeslundi, S. mitis, R. dentocariosa, A, israeli, many subspecies of Fusobacteria, Haemophilus, Campylobacter, Bacteroides melaninogenicus, Asaccharolyticus, anaerobic vibrios, various corroding bacteriodes species, Eikenella corrodens, and gram-negative capnophililic organisms. These microorganisms, in their continuing compulsion to flourish and propagate, deliver a steady stream of enzymes, endotoxins and exotoxins, which impinge on human tissues and severely compromise their normal metabolic functions.

Gingival and marginal periodontal tissue responses to this assault of bacterial antigens lead to inflammation not unlike that which develops in other parts of the body. The inflammatory responses are bodily mechanisms intended to protect tissues, but in fact, they may trigger a chain of catabolic processes leading to tissue damage that is even more severe than that which the bacterial products alone might produce. Initiating factors include collagenase, produced by B. Melaninogenicus, which may trigger the process of destruction of collagen in periodontal tissue. Hyaluronidase and chondroitin sulfate, produced by various diptheroids, can start the destruction of amorphous ground substance. As tissue reacts to protect itself from these toxic assaults, complex changes occur in the immune system, in the function of osteoclasts, in the activity of lymphocytes in the blood streams and in other bodily defenses. Complement activation and the balance of complement-sufficient to complement-deficient serum can lead to increased prostaglandin formation, which in turn provokes the insidious process of alveolar bone resorption. As bone is resorbed into bodily tissues, teeth loosen in their sockets and even tually exfoliate. Loss of first the alveolar bone and then teeth is considered to be the most critical and destructive consequence of inflammatory periodontal disease.

It has been only in the recent past that the importance of the prostaglandins and related compounds as mediators of tissue response in inflammatory processes has started to come into sharp focus. Prostaglandins and related compounds are principally formed by bodily cells at the site of injury by a process known as the arachidonic acid cascade in which certain essential fatty acids, primarily linoleic acid, are enzymatically converted into arachidonic acid, which in turn is further metabolized into leukotrienes, prostacyclins, thromboxanes and prostaglandins. These metabolic end-products are among the most prevalent

autocoids, or internal secretions, carried by the blood stream of mammalian species from one bodily site to another. The pharmacokinetic effects of the prostaglandins and related compounds are profound; they can cause hypotension and vascular dilatation, decreased cell fragility, excite or depress the central nervous system, exert significant effects on the body's endocrine system and on the bony structure, which can lead in turn to rheumatoid arthritis, osteoarthritis and resorption of bone.

Arachidonic acid is a 20-carbon unsaturated fatty acid, derived from linoleic or linoleinic acid, and has 4 double bonds. Chemically, it is 5,8,11,15-eicosatetraenoic acid. When identified by the nomenclature system of numbering carbons from the end of the molecule furthest from the carboxyl group, it is an omega-6 polyunsaturated fatty acid (20:4 n 6). Arachidonic acid is enzymatically converted by three enzymes: (1) phospholipase which gives rise to tissue phosphlolipids, essential components of bodily fatty acid metabolism; (2) lipoxygenase, which produces 20-carbon hydroxy fatty acids and then leukotrienes, damaging to normal cell function; and (3) cyclooxygenase, which give rise to prostaglandins, endoperoxides, thromboxanes and prostaglandins, all of which may also cause disruption of normal body metabolism. The arachidonic acid derivatives believed to be most destructive to dental tissues are the leukotrienes and prostaglandins. Goodson et al. in 1974 (Goodson, J., Dewhirst, F. and Brunetti, A. "Prostaglandin $E_2$ Levels and Human Periodontal Disease," Prostaglandins 6:51-55, 1974), and El Attar in 1976 (El Attar, T. "Prostaglandin $E_2$ in Human Gingiva in Health and Disease and Its Stimulation by Female Sex Steroids", Prostaglandins 11:331-334, 1976) reported higher levels of prostaglandins in the gingival tissues of periodontal disease sufferers than were found in the tissues of those with healthy gingiva. In 1983, El Attar (El Attar, T., and Lin, H., "Relative Conversion of Arachidonic Acid Through Lipoxygenase and Cyclooxygenase Pathways By Homogenates of Diseased Periodontal Tissues", J. Oral Path. 12:7-10, 1983) reported that the cyclooxygenase and lipoxygenase pathways were principal routes of arachidonic acid metabolism in the gingiva of periodontal disease patients.

Chemical agents are known which can interfere with the lipoxygenase and/or cyclooxygenase metabolism of the arachidonic acid cascade. Among these might be mentioned feprazone, aspirin, indomethacin, flurbiprofen, fluocinonide, phenylbutazone, prednisolone, various phenolic compounds and others. Some of these have been evaluated for their effectiveness in periodontal disease therapy. Jeffcoat et al. (Jeffcoat, M., Williams, R., Wechter, W., Johnson, Kaplan M., Gandruf, J. and Goldhaber, P. "Flurbiprofen Treatment of Periodontal Disease in Beagles", J. Periodont. Res. 21:624-633, 1986) adminstered systemic doses of flurbiprofen to beagle dogs with evidence of naturally occurring periodontal disease over a 12-month treatment period and observed a significant reduction in alveolar bone loss as compared to control groups of beagles either left untreated or treated by periodontal surgery. Vogel et al. (Vogel, R., Schneider, L. and Goteiner, D. "The Effects of a Topically Active Non-Steriodal Anti-Inflammatory Drug on Ligature-Induced Periodontal Disease in the Squirrel Monkey", J. Clin. Periodont. 13:139-144, 1986) reported that a particular non-steroidal, anti-inflammatory drug (a substituted oxazolo-pyridine derivative) inhibited gingival bleeding in squirrel monkeys with ligature-induced periodontal disease. Feldman et al. (Feldman, R., Szeto, B., Chauncey, H. and Goldhaber, P., "Non-Steroidal Anti-Inflammatory Drugs in the Reduction of Human Alveolar Bone Loss", J. Clin. Periodont. 10:131-136, 1983) conducted a retrospective evaluation of adult dental patients who had been on long-term therapy with aspirin and/or indomethacin, both of which are prostaglandin inhibitors, and observed a lower rate of alveolar bone resorption than was observed in a matched but undosed control group.

A similar finding had been reported by Waite et al. (Waite, J., Saxton, C., Yong, A., Wagg, B. and Corbett, M., "The Periodontal Status of Subject Receiving Non-Steroidal Anti-Inflammatory Drugs", J. Periodont. Res. 16:100-108, 1981) with respect to gingival inflammation among subjects who had been receiving non-steroidal anti-inflammatory drugs as compared to a matched group who had not received such therapy.

It is the object of this invention to provide new chemical agents and compositions to inhibit the inception and/or progression of periodontal disease in both animals and humans. The formulations permit one to localize and concentrate the effects of such agents directly on the sites of gingival inflammation and periodontal disease involvement by incorporating the agent(s) into vehicles with a strong and persistent tenacity to the oral mucous membranes, from which compositions the treating agent will slowly migrate and diffuse into gingival tissues and thus provide a protracted and consistent reservoir of therapy. These and other objects of the invention will become apparent to those skilled in this art from the following description.

## DESCRIPTION OF THE INVENTION

The present invention includes a process for treating or inhibiting the progression of periodontal disease

utilizing a periodontal disease inhibiting effective amount of a derivative of (3-benzoylphenyl) alkanoic acid preferably compounded into a vehicle which has a strong and continuing adherence to the oral gingival mucosa, and then applying such compositions to the gingival tissues in order to achieve a protracted topical, therapeutic effect for two hours or longer. Effective amounts of the derivative of the (3-benzoylphenyl) alkanoic acid in human gingival tissues generally range from about $10^{-5}$ to $10^{-12}$M, preferably about $10^{-9}$ to $10^{-10}$M. Compositions of preferred vehicles which have acceptable properties are described herein as "mucosal-tenacious" and may be created from a variety of water-soluble or water dispersible polymeric materials combined with other adjuvants.

All such materials used in the vehicle however, must have certain properties in common which are summarized below:

(1) They must be virtually non-toxic systemically.

(2) They must not irritate or damage bodily tissues at the site of the application.

(3) They must be water-soluble or water-dispersible polymeric molecules.

(4) They must be chemically and physically compatible with the (3-benzoylphenyl) alkanoic acid derivative.

(5) They must have a strong and persistent adherence to oral mucosal tissues, preferably for a minimum of 2 hours after application to effected tissues.

(6) They must allow the slow diffusion of the (3-benzoylphenyl) alkanoic acid derivative(s) from the vehicle so that it can contact and permeate the mucosa at the site of application for protracted periods of time.

(7) They must be readily removable from the site of application by use of mild mechanical abrasion and a non-toxic detergent solution.

The mechanism by which a polymeric material bonds to oral mucosal tissues is complex. It is believed that chemical, physical and mechanical bonds form as permeation of molecules takes place into the irregularly contoured surface of the mucosal substrate. Since all body cells in vertebrate animals carry a net negative surface charge and most polymeric agents carry a net positive charge, an electrostatic bond develops due to coulombic attractions, van der Waal forces, hydrogen bonding and covalent bonding.

There are a number of polymeric agents which can be employed to prepare mucosal-tenacious vehicles with the seven required attributes enumerated above. Among these are natural gums, plant extracts, animal extracts, cellulose derivatives, polyvinyl alcohols, polyvinylpyrrolidone, polycarbophil, polyacrylic acid derivatives, polyacrylamides, ethylene oxide homopolymers, polyethylene-polypropylene copolymers, polyethylenimines and others.

When preparing such compositions, it is desirable to use proportions of agents as are identified in the following, all proportions being in parts by weight:

| | |
|---|---|
| A. (3-benzoylphenyl) alkanoic acid derivative(s) | about 0.2-5 parts, preferably about 2-3 parts |

| | |
|---|---|
| B. Mucosal-tenacious polymeric agent | about 2-99 parts, preferably about 40-60 parts |
| C. Formula adjuvants including but not limited to flavor, color, sweetener, preservatives, opacifiers, hydrophobic agents, tissue penetration enhancers, thickeners, wetting agents, fillers, glycerin, propylene glycol, polyethylene glycols and other pharmaceutical necessities. | about 1-90 parts, preferably about 10-50 parts |
| D. Water | about 0-95 parts, preferably about 0.1-9.0 parts |

The (3-benzoylphenyl) alkanoic derivatives used in this invention correspond to the general formula:

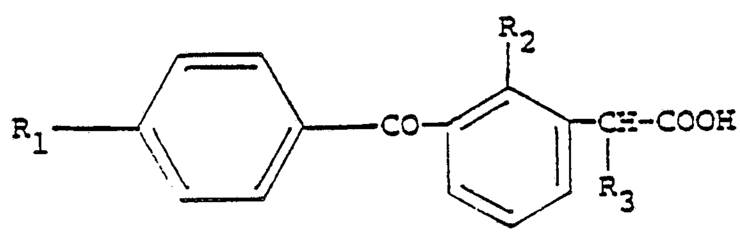

wherein $R_1$ is hydrogen or an alkyl group of 1 to 4 carbons, $R_2$ is hydrogen or alkylthio of 1 to 4 carbons and $R_3$ is a hydrogen or hydroxy group. The (3-benzolyphenyl) alkanoic acid derivatives includes the D- and L-isomers, as well as the alkali metal, ammonium, amine or alkaline earth metal salts of the carboxylic acid moieties of any of these molecular structures. The synthesis of such materials is described in U.S. Patent 3,641,127 and at least one such derivative is used for the treatment of rheumatoid arthritis under the generic name of ketoprofen ($R_1$ and $R_2$ are hydrogen and $R_3$ is a methyl group). However, continued administration for the purpose of treating rheumatoid arthritis can result in troublesome side effects such as nausea, dizziness, blurred vision, gastrointestinal irritation, headache and drowsiness in approximately 15% of users and the necessity for withdrawal of the drug in about 5%. On the other hand, topical use of the same active agent in a composition of a mucosal-tenacious vehicle results in a much lower incidence of side effects. Furthermore, in the intimate contact of the mucosal-tenacious vehicle and the (3-benzoylphenyl) alkanoic acid derivative permits the slow migration of the active ingredient directly into affected tissues for a protracted and continuing period of time so that the continuing inhibition of arachidonic metabolism into destructive end products can take place.

It is important in selecting a composition for the mucosal-tenacious vehicle that it allow the slow

diffusion of the (3-benzoylphenyl) alkanoic acid derivative from the vehicle and into contact with the gingival tissues so that it can be absorbed into those tissues where it will induce its beneficial effects.

The chemical structures of the polymeric agents selected for use in the mucosal-tenacious vehicle of this invention are not nearly as important as their physical properties and ability to satisfy the seven conditions set forth above. However, a large number of materials can be selected which do satisfy these criteria if properly compounded at suitable concentrations into a vehicle such as a semi-solid paste, gel, liquid, ointment or film.

Among such agent are a number of natural hydrophilic polymeric agents, which are enumerated below:

(1) Agar, which is a hydrophilic colloid extracted from certain algae. It is relatively insoluble in cold water but soluble in hot water.

(2) Algin is derived from a brown algae, principally microcystitis pyriera. It is a linear polymer of high molecular weight; it is extracted principally as alginic acid and readily forms water-soluble alkali metal derivatives, amine derivatives and esters, all of which can be used in accordance with the teachings of this invention.

(3) Carageenan is another algae-derived water-soluble polymer and exists principally as the lambda, kappa and iota isomers.

(4) Other water-soluble polymers also derived from marine algae include fucoidan, laminoran and furcellaran.

(5) Gum arabic, also commonly called gum acacia, is the dried gummy exudate of the acacia tree, indigenous to Africa, India, Central America and Southwest North America. It readily forms coacervates with gelatin.

(6) Gum ghatti is another tree exudate which has a higher viscosity in aqueous solutions than gum arabic.

(7) Gum karaya is a tree exudate with a high potential for water absorption and a relatively low pH. At concentrations of 5-20%, it is a strong wet adhesive.

(8) Gum tragacanth is widely used in food processing and is obtained from a perennial shrub found in the near East.

(9) Guar gum is obtained from the guar plant in India and Pakistan and forms viscous, colloidal dispersions in water.

(10) Locust bean gum is derived from the fruit of the carob tree, an evergreen found principally in Southern Europe.

(11) Other natural gums derived from trees and shrubs include quince seed gum, psyllium seed gum, flax seed gum, okra gum and tamarind seed gum.

(12) Pectin is a general term for a group of water-soluble and water-dispersible polysaccharides present in the cell walls of all plant tissues.

(13) A relatively recent type of water-soluble, natural polymer is that produced as an extracellular polysaccharide by bacteria or fungi. Included among these are xanthan gum, polysaccharide Y-1401, scleroglucan and various dextrans.

There are also some starch derivatives which meet many of the criteria outlined for a mucosal-tena cious, water-soluble or water-dispersible polymer above, but are not usable in this invention because of their susceptibility to amylolytic degradation from the enzyme ptyalin found in saliva.

In addition to the natural hydrophilic polymers, synthetic polymers may also be used:

(1) Chemical modification of cellulose provides many derivative which are useful within the teachings of this invention. Among these are methyl cellulose, sodium carboxymethylcellulose, hydroxypropylmethylcellulose, hydroxypropylethylcellulose, hydroxypropyl cellulose; and ethylhydroxyethyl cellulose. These agents can be prepared in a wide range of solubility and viscosity ranges.

(2) Polyvinyl alcohol is produced by the alcoholysis of polyvinyl acetate and can be made in a number of molecular weights, water-solubility ranges and viscosity ranges.

(3) Polyvinylpyrrolidone is a homopolymer of N-vinylpyrrolidone with a high level of water solubility and pronounced viscosity-building properties.

(4) Polyacrylic acid derivatives can be used directly but more often are used with other copolymers; an important polyacrylic acid copolymer is polycrabophil.

(5) Particularly useful materials are the partial calcium/sodium salts of lower alkyl vinyl-maleic acid anhydride copolymers, sold commercially as "Gantrez" and "Ucarset".

(6) Polyacrylamide is a polymer of acrylamide and can be polymerized or copolymerized by a variety of synthetic approaches.

(7) Ethylene oxide polymers of very high molecular weight are commercially sold by the Union Carbide Co. as water-soluble resins ("Polyox"). They range in molecular weight from a few hundred

thousand to five million or more. The higher molecular weight derivatives have extraordinary viscosity-building effects in water and other solvents, as well as pronounced mucosal-tenacity.

(8) Polyethylenimines are produced from the monomer ethylenimine in the presence of an acid catalyst. They are of special interest for adherent formulations because of their tendency to form strong electrostatic bonds.

It is also possible to use at least one material of animal origin:

(1) Gelatin is a partially hydrolyzed protein derived from the skin, connective tissues and bones of mammalian animals; that derived by acid treatment is Type A and that from alkali treatment is Type B.

These various polymeric materials herein described are illustrative of the many agents from which a composition can be compounded into useful mucosal-tenacious vehicles. They may be used singly or in combination, in a wide range of concentrations, and in the presence of many other agents intended to control rates of water absorption and swelling, ingredients to enhance tissue penetration, various fillers, buffers, sweeteners, flavors, bodying agents and other pharmaceutical necessities.

Further modifications of the mucosal-tenacious vehicle and the molecular structure of modified (3-benzyolyphenyl) alkanoic acid derivatives may be made by those skilled in the art without departing from the teaching of this invention. Also, while less preferred, it will be appreciated that any other type of conventional topical formulation can be used as the active ingredient carrier. For example, a variety of carrier compositions are described in U.S. Patent 4,615,697.

Examples 1-6 below are illustrative of some mucosal-tenacious vehicles with a (3-benzoylphenyl) alkanoic acid derivative which can be used in accordance with teachings of this invention. Example 7 demonstrates the pronounced tenacity to mucosal tissues of typical compositions exemplified herein, and Example 8 documents the superior properties of (3-benzoylphenyl) alkanoic acid derivative in inhibiting periodontal prostaglandin formation.

Example 1

A composition of matter is prepared in accordance with the following formula:

| | |
|---|---|
| Karaya Gum | 51.0% |
| Petrolatum | 38.6% |
| Mineral oil | 7.2% |
| Magnesium oxide | 1.0% |
| (3-benzoylphenyl) alkanoic acid derivative | 2.0% |
| Formula adjuvants (color, flavor) | 0.2% |

The karaya gum is a strong mucosal-tenacious, natural polymeric hydrocolloid; the petrolatum and mineral oil are hydrophobic agents which further improve the resistance of the vehicle to rapid leaching and removal of the vehicle from the site of mucosal application and also provides plasticity and ready extrudibility of the mucosal-tenacious mixture from collapsible tubes; the magnesium oxide is an opacifier and alkaline buffer for the normally mildly acidic karaya gum; an oil-soluble pigment and flavor are for cosmetic purposes; and the (3-benzoylphenyl) alkanoic acid derivative is an inhibitor of arachidonic acid metabolism into prostaglandins, thromboxanes, leukotrienes and endoperoxides.

Example 2

Another useful composition is a mucosal-tenacious film prepared in accordance with the following formula:

| | |
|---|---|
| Ethylene oxide homopolymer of approximately 3,000,000 MW | 30.0% |
| Polyvinylpyrrolidone | 50.0% |
| Polyethylene glycol 4000 | 15.0% |
| Glycerin | 2.0% |
| (3-benzoylphenyl) alkanoic acid derivative | 3.0% |

The first four ingredients are comminuted into an intimate mixture and warmed to 40° C at which time the (3-benzoylphenyl) alkanoic acid derivative is incorporated and mixed thoroughly.

The final mixture is cooled to about 25° C and then extruded through stainless steel rollers into a film approximately 2 mm thick. After further cooling, the extruded film is cut by knives into rectangular strips of approximately 2.0 x 0.5 cm in dimension. In order to use the strips for the treatment of periodontal disease, they are moistened with water and pressed firmly onto the gingival mucosa at the site of treatment. It is desirable but not essential to cover one side of the mucosal-tenacious strip during manufacture with a water-impermeable cover film so that the mucosal-tenacious composition is protected from other oral tissues such as the tongue, while still allowing its intimate contact with the gingival mucosa.

Example 3

A composition suitable for insufflation into periodontal pockets more than 3 mm deep can be prepared as follows:

| | |
|---|---|
| Sodium carboxymethylcellulose (< 100 mesh average particle size) | 90.0% |
| Polyethylene glycol 6000 (< 100 mesh) | 5.2% |
| (3-benzoylphenyl) alkanoic acid derivative (< 100 mesh) | 4.8 |

The components are intimately mixed and ground to a fine particle size in an air-attrition mill. When introduced directly into periodontal pockets by insufflation, the polyethylene glycol acts as a hydrophilic attractant for intersulcular fluid and other aqueous materials such as saliva or ingested fluids. As water is attracted to and held by the mixture, the sodium carboxymethylcellulose in the ve hicle is wetted and swelled and adheres tenaciously to the mucosal walls of the periodontal pocket. This swollen gel persists for hours and continues to provide a continuing release of the (3-benzoylphenyl) alkanoic acid derivative. Other cellulose derivatives such as hydroxyethylcellulose, methylcellulose, hydroxypropylcellulose, and hydroxyethylpropylcellulose may be substituted for the sodium carboxymethylcellulose.

Example 4

A liquid composition, also suitable for installation into periodontal pockets, may be prepared as follows:

| | |
|---|---|
| Lower alkyl vinyl ether-maleic acid anhydride copolymer | 20.0% |
| Pectin gum | 10.0% |
| Gelatin | 10.0% |
| Liquid petrolatum | 53.0% |
| Polyethylene powder | 5.0% |
| (3-benzoylphenyl) alkanoic acid derivative | 2.0% |

All ingredients are mixed thoroughly and reduced in particle size by use of a comminuting mill. The resulting product is a viscous suspension which can be delivered into periodontal pockets by use of a blunt-end hypodermic needle affixed to a plunger-type syringe or other suitable dispensing device. Under the influence of intrasulcular fluid and other intrinsic and extrinsic aqueous materials, the first three ingredients in the formula swell and adhere strongly to inner mucosal wall of the periodontal pocket. The (3-benzoylphenyl) alkanoic acid derivative is released slowly from the heavy gel-like tenaciously-adherent suspension and effectively blocks the formation of arachidonic acid metabolites within the diseased tissues.

Example 5

Another useful cream-like composition is prepared in accordance with the following formula:

| | |
|---|---|
| Petrolatum | 30.9% |
| Mineral oil | 13.0% |
| "Ucarset" resin | 30.0% |
| Sodium carboxymethylcellulose | 24.0% |
| (3-benzoylphenyl) alkanoic acid derivative | 2.0% |
| Formula adjuvants | 0.1% |

Example 6

Still another liquid suspension of a mixture of synthetic polymers with a (3-benzoylphenyl) alkanoic acid derivative and a pronounced mucosal tenacity is exemplified as follows:

| | |
|---|---|
| Sodium carboxymethylcellulose | 23.0% |
| Polyethylene oxide homopolymer | 17.0% |
| Polyethylene powder | 1.5% |
| (3-benzoylphenyl) Alkanoic acid derivative | 2.0% |
| Mineral oil | 56.0% |
| Formula adjuvants | 0.5% |

Example 7

The following example provides evidence of the pronounced mucosal tenacity of typical preparations characterized in this invention. Mucosal tenacity was determined by an in vitro procedure developed to determine the persistence of materials to mucosal tissue.

Sections of the fresh oral mucosa from beef cattle were obtained and brushed gently with a soft-bristled toothbrush and a mild detergent solution to remove extraneous debris. Sections measuring approximately 5 x 10 cm were cut with a straight edge and a razor blade while being kept moist. Separate pieces were wrapped around ordinary glass microscope slides and held in place by taut, elastic rubber bands. Each separate tissue-covered slide was then spread with a layer approximately 1 mm thick of an oral treatment product, with each product containing 2% of a (3-benzoylphenyl) alkanoic acid derivative. Each slide as weighed in the moist state before and after application of the treating agent. Slides were then attached by rubber bands to the baskets of a USP tablet disintegration apparatus. The beaker into which the baskets and slides with oral mucosa covered with the mucosal-tenacious compositions were alternately lowered and raised into a bath of physiological saline maintained at 37° C. At periodic intervals, the slides were removed from the basket and reweighed. The following table demonstrates the persistence of mucosal-tenacious compositions as described herein.

| Composition | Percentage of Original Amount Retained at Time in Hours | | | |
|---|---|---|---|---|
| | 0 | 2 | 4 | 6 |
| Example 1 of this invention | 100.00 | 94.6 | 94.0 | 88.0 |
| Example 5 of this invention | 100.0 | 78.2 | 50.6 | 50.4 |

As can be observed, typical mucosal-tenacious compositions as described herein were highly resistant to aqueous leaching with physical agitation for periods in excess of 6 hours, a period sufficiently long for the active ingredient to exert its desired therapeutic effects.

Example 8

This example documents the remarkable ability of a (3-benzoylphenyl) alkanoic acid derivative to inhibit the cyclooxygenase enzymatic convention of arachidonic acid into tissue-destructive prostaglandins.

Inflamed gingival tissues from the mouths of humans suffering from periodontal disease were used fresh or stored in liquid nitrogen until used. Tissues were pooled, homogenized in 0.2 M TRIS buffer, and centrifuged. Radiolabeled arachidonic acid ($^{14}C$) was added to the centrifrugate into which various inhibiting agents under study had been incorporated into separate aliquots, at various molar levels (log range of $10^{-3}$ to $10^{-9}$ M). The reaction between arachidonic acid and test materials was carried out for 2 hours at 37° C.

Prostaglandins in the biological reaction mixtures were extracted and quantitatively determined by high performance liquid chromatography. $PGE_2$ was found to be the major ($^{14}C$) end product of cyclooxygenase enzymic activity. A number of materials which have been reported to be prostaglandin inhibitors were compared. The following table indicates the molar strength of test materials required to inhibit 50 percent of the prostaglandin level produced by arachidonic acid used alone:

| Test Material | $IC_{50}$ |
|---|---|
| 2-methyl (3-benzoylphenyl) propanoic acid (ketoprofen) | $5.4 \times 10^{-9}$ |
| Alpha tochoperol | $7.0 \times 10^{-9}$ |
| Indomethacin | $1.0 \times 10^{-8}$ |
| Flurbiprofen | $1.5 \times 10^{-8}$ |
| Meclofenamate | $1.5 \times 10^{-6}$ |
| Naproxen | $2.5 \times 10^{-6}$ |
| Docosahexaenoic acid | $1.0 \times 10^{-5}$ |
| Eicosapentaenoic acid | $1.5 \times 10^{-5}$ |
| Ibuprofen | $1.5 \times 10^{-5}$ |

It can be observed that the (3-benzoylphenyl) alkanoic acid was slightly more effective than alpha tocopherol, and many times more effective than indomethacin or ibuprofen.

Without any intention of being limited to theory, it is believed that the mechanism by which the (3-benzoylphenyl) alkanoic acid derivative functions in inhibiting the destructive activity of the prostaglandins is by attaching to the prostaglandin molecule and thus blocking its binding with bodily cells, or by binding directly to bodily cells so that the prostaglandins cannot readily attach to them.

**Claims**

1. A method for the treatment of periodontal disease which comprises the step of topically applying a periodontal disease inhibiting effective amount of a derivative of (3-benzoylphenyl) alkanoic acid of the formula:

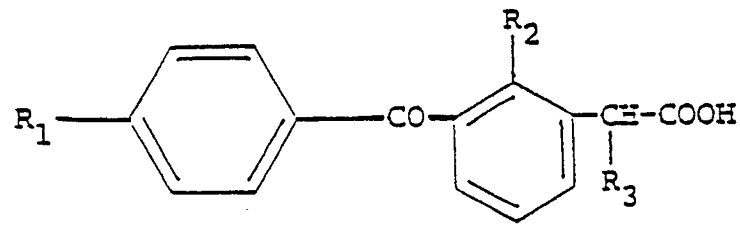

wherein $R_1$ is hydrogen or alkyl of 1 to 4 carbon atoms, $R_2$ is hydrogen or alkylthio of 1 to 4 carbon atoms and $R_3$ is a hydrogen or a hydroxy group, and the pharmaceutically acceptable salts thereof.

2. The method of claim 1, in which $R_1$ and $R_2$ are hydrogen and $R_3$ is methyl.

3. The method of claim 2, in which the amount is from about $10^{-5}$ to $10^{-12}$ M.

4. The method of claim 3, in which the amount is about $10^{-9}$ to $10^{-10}$ M.

5. The method of claim 1, in which the amount is about $10^{-5}$ to $10^{-12}$ M.

6. The method of claim 5, in which the amount is about $10^{-9}$ to $10^{-10}$ M.

7. The method of claim 1, in which said derivative is incorporated within a mucosal-tenacious carrier.

8. The method of claim 7, in which said mucosal-tenacious carrier comprises a water soluble or water dispersible polymer.

9. The method of claim 8, in which said combination of derivative and carrier contains about 0.0-5 parts derivative, about 2-99 parts mucosal-tenacious polymer and about 0.1-90 parts adjuvant.

10. The method of claim 9, in which $R_1$ and $R_2$ are hydrogen and $R_3$ is methyl.

11. The method of claim 10, in which said mucosal-tenacious polymer is selected from the group consisting of karaya gum, ethyleneoxide polymer, sodium carboxymethylcellulose and lower alkyl vinyl ether-maleic acid anhydride copolymer.

12. The method of claim 8, in which said mucosal-tenacious polymer is selected from the group consisting of karaya gum, ethyleneoxide polymer, sodium carboxymethylcellulose and lower alkyl vinyl ether-maleic acid anhydride copolymer.

European Patent Office

# EUROPEAN SEARCH REPORT

Application Number

EP 89 31 0283

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | PATENT ABSTRACTS OF JAPAN vol. 8, no. 241 (C-250)(1678), 6 November 1984; & JP - A - 59 122422 (TOUKOU YAKUHIN KOGYO K.K.) 14.07.1984 --- | 1,2,7,8 ,10-12 | A 61 K 7/16<br>A 61 K 7/24<br>A 61 K 31/19 |
| A | WO-A-8 803 021 (THE UPJOHN CO.) * whole document * --- | 1-12 | |
| A | FR-A-2 315 948 (GABA AG) * whole document * --- | 1-12 | |
| A | FR-A-2 258 865 (THE PROCTER & GAMBLE CO.) * claims * ----- | 1-12 | |
| | | | TECHNICAL FIELDS SEARCHED (Int. Cl.5) |
| | | | A 61 K |

The present search report has been drawn up for all claims

| Place of search | Date of completion of the search | Examiner |
|---|---|---|
| BERLIN | 11-12-1989 | SIATOU E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)